# EUROPEAN PATENT APPLICATION

(11) **EP 2 767 950 A2**
(43) Date of publication of application: **20.08.2014**
(21) Application number: 12840418.3
(22) Date of filing: 10.10.2012
(51) Int. Cl.: G06Q 50/22

(54) **AUTOBOX MANAGEMENT SYSTEM**

(30) Priority: 10.10.2011 KR 20110103298
(71) Applicant: JVM Co., Ltd., Dalseo-gu Daegu 704-900 (KR)
(72) Inventor: KIM, Jun-Ho, Daegu 706-819 (KR)
(74) Representative: Von Kreisler Selting Werner - Partnerschaft von Patentanwälten und Rechtsanwälten mbB
(86) International application number: PCT/KR2012/008224
(87) International publication number: WO 2013/055109

(57) **Abstract**

Disclosed is an autobox management system to register intrinsic ID information in an autobox, which stores drugs therein, thereby determining if the autobox is original when the autobox is mounted in a cabinet of a hospital, and to manage information of drug consumption through the communication with a cabinet, a hospital server, and a producer server. The autobox management system including a production management unit to produce an autobox, individually register autobox ID information in the autobox, and authenticate that the autobox is original, and a hospital management unit to request determination on if the autobox is original and to transmit information of drug consumption to the production management unit through communication with the production management unit.

## Description

### [Technical Field]

The present invention relates to a technology of managing an autobox to receive drugs therein. In more particular, the present invention relates to an autobox management system capable of registering intrinsic ID information in an autobox, which can store drugs therein, to determine if the autobox is original when the autobox is mounted in a cabinet of a hospital, and capable of managing information of drug consumption through the communication with a cabinet, a hospital server, and a producer server.

### [Background Art]

In general, when drugs are prepared through a hand of a person in various places such as hospitals or large pharmacies to manage and prepare drugs in large quantities, a considerable time may be spent and many errors may occur, thereby causing a serious damage to the health of a patient.

Accordingly, many systems have been developed to search for drugs according to prescription information and to distribute the drugs according to a dose to be taken or injected

The automation of the drug preparation work can reduce the waiting time of a patient, save personnel expenses, and lower medication errors caused by errors in the drug preparation work.

In an automatic drug distribution system according to the related art, an autobox to store and discharge drugs is mounted with an embedded memory having the information of the drugs in a cabinet.

In other words, according to the related art, since the information of the drugs is registered in the memory of each autobox, the information of the drugs must be newly written in the memory when the autobox is replaced with new one. Accordingly, the autobox may not be easily replaced.

Further, as many data are stored in the memory of the autobox, the communication efficiency between a cabinet and a hospital server may be degraded.

In addition, there is not provided a unit to determine if the autobox is original, so that the medication errors resulting from the illegal use of the autobox may occur.

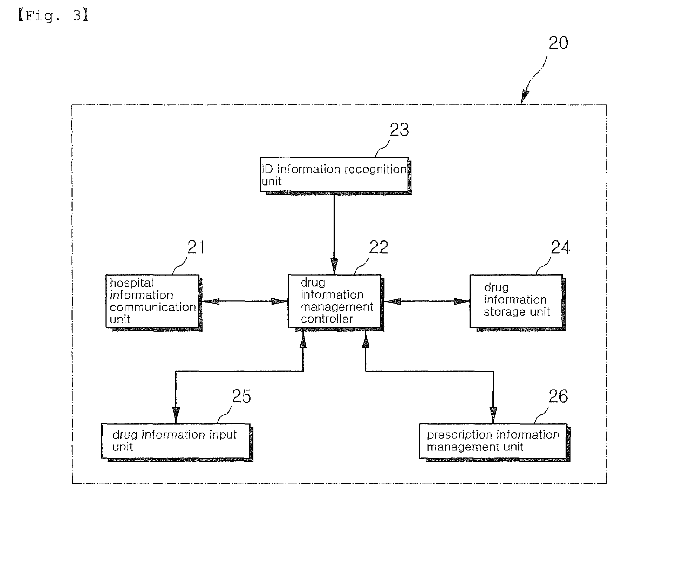

The related art of the present invention includes a drug collection system disclosed in Korea Patent Registration No. 10-0605769 (issued on July 20, 2006; see line 8-16, page 5, and FIG. 3). The present invention provides an autobox management system capable of determining if an autobox is original and managing the information of drug consumption through the communication among a cabinet, a hospital server, and a manufacture server when a cabinet is mounted.

### [Disclosure]

### [Technical Problem]

The present invention has been made keeping in mind the above problems occurring in the related art, and an objective of the present invention is to provide an autobox management system capable of facilitating the management of an autobox and the replacement and the reuse of the autobox by assigning intrinsic ID information to the autobox to store drugs and discharge the drugs according to prescription information.

Another objective of the present invention is to provide a cabinet to transmit the information of an autobox mounted in the cabinet to a hospital management unit in real time, and manage the discharge situation of drugs.

Another objective of the present invention is to provide a production management unit to create intrinsic autobox ID information, individually register the autobox ID information in the autobox, and manage information of the autobox depending on the autobox ID information.

Another obj ective of the present invention is to additionally provide a production information search unit and an original verifying unit to determine if the autobox is original by using the autobox ID information transmitted from a hospital management unit.

Another objective of the present invention is to provide a production information communication unit to receive drug discharge information of an autobox.

Another object of the present invention is to additionally provide a drug consumption analysis unit to variously analyze the consumption of drugs resulting from the drug discharge of an autobox according to hospitals, regions, and terms.

Another object of the present invention is to provide a hospital management unit to recognize autobox ID information, determine if an autobox is original, and manage information introduced into the autobox.

Another object of the present invention is to provide a prescription information management unit to transmit prescription information to a cabinet.

Another obj ect of the present invention is to provide a cabinet to manage an autobox mounted in the cabinet and discharge drugs from the autobox according to the prescription information.

Another object of the present invention is to provide a cartridge to individually mount an autobox in a cabinet and identify the autobox corresponding to each position.

### [Technical Solution]

There is provided an autobox management system including a production management unit to produce an autobox, individually register autobox ID information in the autobox, and authenticate that the autobox is original, and a hospital management unit to request determination on if the autobox is original and to transmit information of drug consumption to the production management unit through communication with the production management unit.

In addition, the autobox management system further includes a cabinet to transmit the autobox ID information and drug discharge situation information according to mounting of the autobox through communication with the hospital management unit.

In the autobox management system, the production management unit includes a production information communication unit to receive the autobox ID information, which is registered in the autobox, from the hospital management unit and to transmit information on whether the autobox is original or not, a production information management controller connected with the production information communication unit to manage the autobox ID information according to production of the autobox, an ID information generating unit connected with the production information management controller to generate the autobox ID information, an ID information registration unit connected with the production information management controller to register the autobox ID information generated from the ID information generating unit in an internal memory of the autobox, and a production information storage unit connected with the production information management controller to store the autobox ID information and history information according to the production of the autobox.

In the autobox management system, the production management unit further includes a production information search unit connected with the production information management controller to search for production information of the autobox by using the autobox ID information, and an original verifying unit connected with the production information management controller to determine if the autobox is original depending on a search result of the production information search unit.

In the autobox management system, the hospital management unit includes a hospital information communication unit to transmit the autobox ID information and information of drug discharge situation from the autobox to the production management unit, and to receive information on whether the autobox is original or not from the production management unit, a drug information management controller connected with the hospital information communication unit to manage the drug discharge situation from the autobox according to information and prescription information of a drug stored in the autobox, an ID information recognition unit connected with the drug information management controller to recognize the autobox ID information registered in the autobox, a drug information input unit connected with the drug information management controller to input the information of the drug introduced into the autobox, and a drug information storage unit connected with the drug information management controller to store the autobox ID information and the information of the drug stored in the autobox.

Further, in the autobox management system, the cabinet includes a cabinet communication unit to transmit the autobox ID information to the hospital management unit and to receive prescription information when the autobox is mounted, a cabinet controller connected with the cabinet communication unit to control driving of the autobox, and a cartridge connected with the cabinet controller to fix the autobox into the cabinet.

### [Advantageous Effects]

As described above, according to the present invention, the autobox management system can facilitate the management of the autobox and the replacement and the reuse of the autobox by assigning the intrinsic ID information to the autobox to store drugs and discharge the drugs according to prescription information.

In addition, the cabinet is provided to transmit the information of the autobox mounted in the cabinet to the hospital management unit in real time, and manage the discharge situation of drugs in real time.

In addition, the production management unit is provided to create intrinsic autobox ID information, individually register the autobox ID information in the autobox, and schematically manage information of the autobox depending on the autobox ID information.

Further, the production information search unit and the original verifying unit are additionally provided to determine if the autobox is original by using the autobox ID information transmitted from a hospital management unit.

Further, the production information communication unit is provided to receive drug discharge information of the autobox in real time.

In addition, the drug consumption analysis unit is additionally provided to variously analyze the consumption of drugs resulting from the drug discharge of an autobox according to hospitals, regions, and terms, so that the analysis can be reflected in the drug production work.

In addition, the hospital management unit is provided to recognize the autobox ID information, determine if the autobox is original, and manage information introduced into the autobox.

Further, the prescription information management unit is provided to transmit prescription information to the cabinet.

Further, the cabinet is provided to manage the autobox mounted in the cabinet and discharge the drugs from the autobox according to the prescription information.

Further, the cartridge is provided to individually mount the autobox in the cabinet and identify the autobox corresponding to each position so that the autobox can be driven.

### [Description of Drawings]

FIG.1 is a block diagram showing the whole structure of an autobox management system according to the present invention.
FIG.2 is a detailed block diagram showing a production management unit in the autobox management system according to the present invention.
FIG.3 is a detailed block diagram showing a hospital management unit in the autobox management system according to the present invention.
FIG.4 is a schematic view showing a cabinet in the autobox management system according to the present invention.
FIG. 5 is a flowchart showing the operation of the autobox management system according to the present invention.

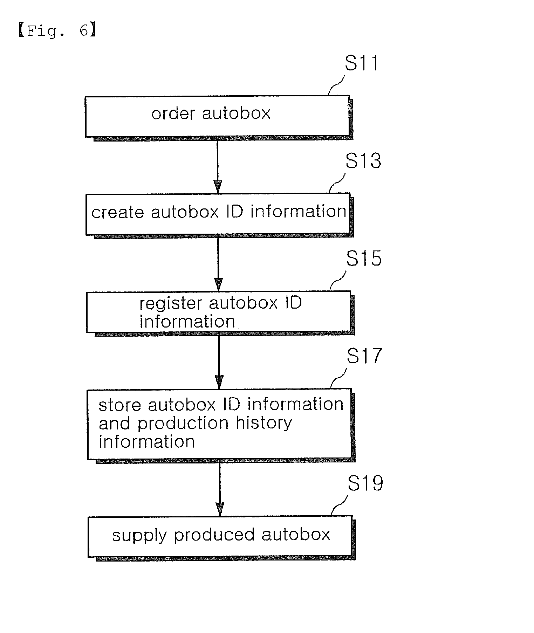

FIG. 6 is a detailed flowchart showing step S10 in an operating method using the autobox management system according to the present invention.

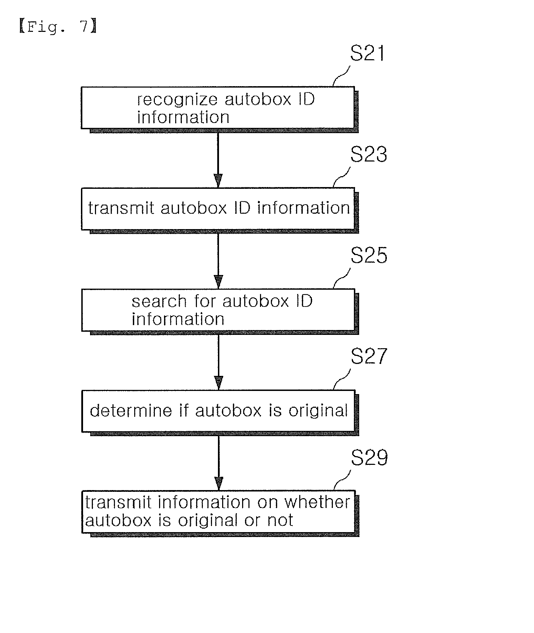

FIG. 7 is a detailed flowchart showing step S20 in the operating method using the autobox management system according to the present invention.

### [Best Mode]

### [Mode for Invention]

Hereinafter, an autobox management system and a method of managing an autobox according to the present invention will be described in detail.

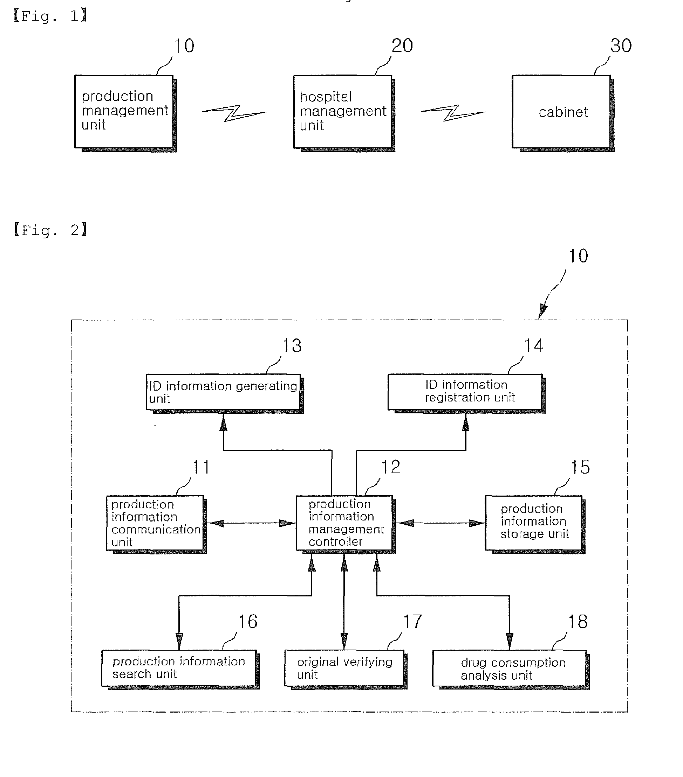

FIG.1 is a block diagram showing the whole structure of an autobox management system according to the present invention including a production management unit 10, a hospital management unit 20, and a cabinet 30.

The production management unit 10 produces the autobox, individually registers autobox ID information in the autobox, and verifies that the autobox is original. As shown in the detailed structure of FIG. 2, the production management unit 10 includes a production information communication unit 11, a production information management controller 12, an ID information generating unit 13, an ID information registration unit 14, a production information production information storage unit 15, a production information search unit 16, an original verifying unit 17 and a drug consumption analysis unit 18.

The production information communication unit 11 receives the autobox ID information, which is registered in the autobox, from the hospital management unit 20 and transmits information on whether the autobox is original or not. The production information communication unit 11 transmits the received autobox ID information to the production information management controller 12.

In addition, the production information communication unit 11 according to the present invention preferably receives information of drug discharge from the autobox from the hospital management unit 20.

The production information management controller 12 is connected with the production information communication unit 11 to manage the autobox ID information according to the order and the production of the autobox.

The ID information generating unit 13 is connected with the production information management controller 12 to generate the autobox ID information.

The autobox ID information according to an embodiment of the present invention is constructed in a hexadecimal number having 16 digits. The autobox ID information serves as intrinsic ID information to individually identify the autobox, and a manufacture creates, stores, and manages the autobox ID information.

The ID information registration unit 14 is connected with the production information management controller 12 to register the autobox ID information generated from the ID information generating unit 13 in an internal memory of the autobox.

In other words, the autobox ID information, which is intrinsic ID information, is registered in the internal memory of each produced autobox.

The production information storage unit 15 is connected with the production information management controller 12 to store the autobox ID information and production history information of the autobox.

The production information search unit 16 is connected with the production information management controller 12 to search for production information of the autobox and to provide the search result to the production information management controller 12.

The original verifying unit 17 is connected with the production information management controller 12 to determine if the autobox is original depending on the search result of the production information search unit 16.

In other words, the original verifying unit 17 is provided to determine if the autobox is original so that the illegal use of the autobox can be prevented.

The drug consumption analysis unit 18 is connected with the production information management controller 12 to analyze a consumption situation of each drug by using information of the drug discharged from the autobox.

The drug consumption analysis unit 18 according to the present invention is provided so that a worker can variously analyze information of the type of drugs received in the autobox and the consumption of the drugs according to hospitals, regions, and terms to reflect the analysis in a drug production work.

The hospital management unit 20 makes communication with the production management unit 10 to request the determination if the autobox is original and to transmit the information of drug consumption to the production management unit. As shown in FIG. 3, the hospital management unit 20 according to the present invention includes a hospital information communication unit 21, a drug information management controller 22, an ID information recognition unit 23, a drug information storage unit 24, a drug information input unit 25, and a prescription information management unit 26.

The hospital information communication unit 21 transmits the autobox ID information and the drug discharge situation information of the autobox to the production management unit 10. The hospital information communication unit 21 receives information on whether the autobox is original or not from the production management unit 10.

The drug information management controller 22 is connected with the hospital information communication unit 21 to manage the drug discharge situation from the autobox according to information and prescription information of a drug stored in the autobox.

The ID information recognition unit 23 is connected with the drug information management controller 22 to recognize the autobox ID information registered in the autobox.

The drug information storage unit 24 is connected with the drug information management controller 22 to store the autobox ID information and the information of drugs stored in the autobox.

The drug information input unit 25 is connected with the drug information management controller 22 to input the drug information introduced into the autobox.

The drug information may include information of names of drugs, the manufacturers of the drugs, the number of the drugs, and the expiration dates of the drugs.

The prescription information management unit 2 6 is connected with the drug information management controller 22 to receive prescription information of a medical diagnosis terminal and to manage the prescription information.

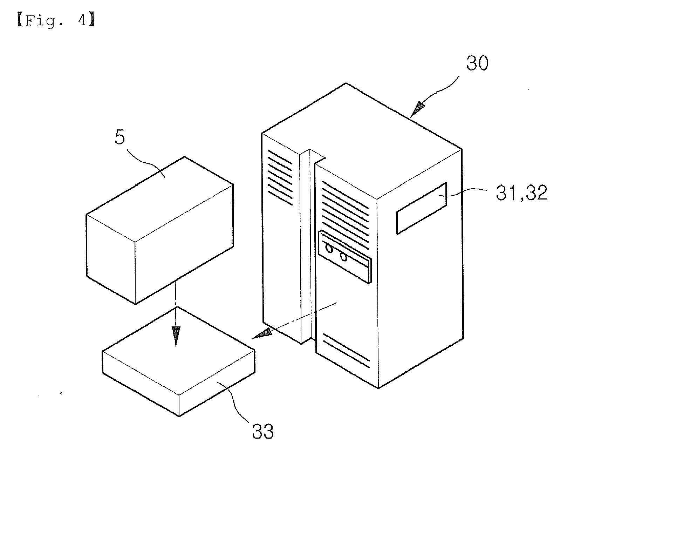

The cabinet 30 makes communication with the hospital management unit 20 to transmit the autobox ID information and the drug discharge situation information as the autobox is mounted in the cabinet 30. The cabinet 30 according to the present invention includes a cabinet communication unit 31, a cabinet controller 32, and a cartridge 33 as shown in FIG. 4.

The cabinet communication unit 31 transmits the autobox ID information to the hospital management unit 20 and receives the prescription information when the autobox is mounted.

The cabinet controller 32 is connected with the cabinet communication unit 31 to control the driving of the autobox.

The cartridge 33 is connected with the cabinet controller 32 to fix the autobox into the cabinet.

In addition, preferably, the cartridge 33 according to the present invention is operated by using ID information in order to recognize a location of the autobox.

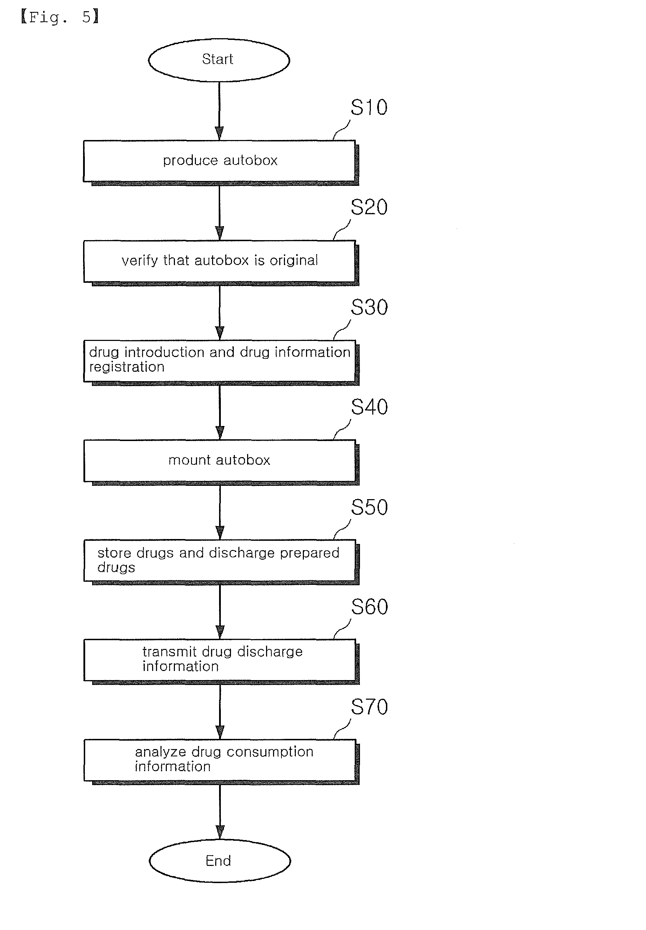

According to an operating method of the autobox management system of the present invention, an autobox is produced by using the production management unit 10 (step S10) as shown in FIG. 5.

According to the step S10, autoboxes are ordered to the production management unit 10 through the hospital information communication unit 21 of the hospital management unit 20 (step S11), and autobox ID information is created according to the number of ordered autoboxes by using the ID information generation unit 13 (step S13).

Next, the autobox ID information, which is generated from the ID information generating unit 13, is registered in an internal memory of the autobox by using the ID information registration unit 14 (step S15), the autobox ID information is stored in the production information storage unit 15 together with the production history information (Step S17), and the autobox having the registered autobox ID information is supplied (step S19).

Thereafter, a determination is made on if the autobox is original by using the hospital management unit 20 (step S20).

According to the step (S20), the autobox ID information registered in the internal memory of the autobox is recognized by using the ID information recognition unit 23 (step S21) and the recognized autobox ID information is transmitted to the production management unit 10 by using the hospital information communication unit 21 (step S23).

Thereafter, the autobox ID information stored in the production information storage unit 15 is searched by using the production information search unit 16 (step S25). The original of the autobox is verified by the original verifying unit 17 based on the matching state of the autobox ID information and the registered history information (step S27). The information of the original state of the autobox is transmitted to the hospital management unit 20 by using the production information communication unit 11 (step S29).

If it is determined that the autobox is not original, measures to restrict the use of the related autobox are preferably taken.

Next, if it is determined that the autobox is original, drugs are introduced into the autobox, and the information of the drugs is input by using the drug information input unit 25 (step S30). The input drug information is stored in the drug information storage unit 24 together with the autobox ID information.

Thereafter, the autobox having the introduced drugs is transferred to the cabinet 30, mounted in the cartridge 33, and inserted into the cabinet 30 (step S40).

If the autobox is inserted into the cabinet 30 in step S40 according to the present invention, the location of the autobox based on the ID of the autobox according to the mounting location of the cartridge 33 is transmitted to the hospital management unit 20.

Next, the prescription information received in the prescription information management unit 26 is transmitted to the cabinet communication unit 31, so that the drugs in the autobox 5 are discharged according to the prescription information (step S50).

Thereafter, the drug discharge situation of the autobox 5 is transmitted to the hospital management unit 20 by using the cabinet communication unit 31 (step S60).

Subsequently, the information of the drug discharge situation of the autobox 5 is transmitted THE production management unit 10 to analyze the information of drug consumption (step S70)

In other words, the information of the drug discharge situation received from the hospital management unit 20 can be variously analyzed according to hospitals, regions, and terms through the drug consumption analysis unit 18. The analysis information can be reflected in the drug production work.

As described above, if the autobox management system according to the present invention is applied, the intrinsic ID information is provided for the autobox to discharge drugs according to the prescription information, thereby facilitating the management of the autobox, facilitating the replacement and the re-use of the autobox, and preventing the autobox from being illegally used.

Although the exemplary embodiments of the present invention have been described, it is understood that the present invention should not be limited to these exemplary embodiments but various autoboxmanagement systems canbe realized by one ordinary skilled in the art within the spirit and scope of the present invention as hereinafter claimed.

## Claims

1. An autobox management system comprising:
a production management unit to produce an autobox, individually register autobox ID information in the autobox, and authenticate that the autobox is original; and
a hospital management unit to request determination on if the autobox is original and to transmit information of drug consumption to the production management unit through communication with the production management unit.

2. The autobox management system of claim 1, further comprising a cabinet to transmit the autobox ID information and drug discharge situation information according to mounting of the autobox through communication with the hospital management unit.

3. The autobox management system of claim 1, wherein the production management unit comprises:
a production information communication unit to receive the autobox ID information, which is registered in the autobox, from the hospital management unit and to transmit information on whether the autobox is original or not;
a production information management controller connected with the production information communication unit to manage the autobox ID information according to production of the autobox;
an ID information generating unit connected with the production information management controller to generate the autobox ID information;
an ID information registration unit connected with the production information management controller to register the autobox ID information generated from the ID information generating unit in an internal memory of the autobox; and
a production information storage unit connected with the production information management controller to store the autobox ID information and history information according to the production of the autobox.

4. The autobox management system of claim 3, wherein the production management unit further comprises:
a production information search unit connected with the production information management controller to search for production information of the autobox by using the autobox ID information; and
an original verifying unit connected with the production information management controller to determine if the autobox is original depending on a search result of the production information search unit

5. The autobox management system of claim 3, wherein the production information communication unit receives information of a drug, which is discharged from the autobox, from the hospital management unit.

6. The autobox management system of claim 5, wherein the production management unit further comprises a drug consumption analysis unit connected with the production information management controller to analyze a consumption situation of each drug by using information of the drug discharged from the autobox.

7. The autobox management system of claim 1, wherein the hospital management unit comprises:
a hospital information communication unit to transmit the autobox ID information and information of drug discharge situation from the autobox to the production management unit, and to receive information on whether the autobox is original from the production management unit;
a drug information management controller connected with the hospital information communication unit to manage the drug discharge situation from the autobox according to information and prescription information of a drug stored in the autobox;
an ID information recognition unit connected with the drug information management controller to recognize the autobox ID information registered in the autobox;
a drug information input unit connected with the drug information management controller to input the information of the drug introduced into the autobox; and
a drug information storage unit connected with the drug information management controller to store the autobox ID information and the information of the drug stored in the autobox.

8. The autobox management system of claim 7 , wherein the hospital management unit further comprises a prescription information management unit connected with the drug information management controller to receive the prescription information of a medical diagnosis terminal and to manage the prescription information.

9. The autobox management system of claim 2, wherein the cabinet comprises:
a cabinet communication unit to transmit the autobox ID information to the hospital management unit and to receive prescription information when the autobox is mounted;
a cabinet controller connected with the cabinet communication unit to control driving of the autobox; and
a cartridge connected with the cabinet controller to fix the autobox into the cabinet.

10. The autobox management system of claim 9, wherein the cartridge is operated by using ID information in order to distinguish locations of autoboxes.
